# EUROPÄISCHE PATENTANMELDUNG

(11) **EP 0 906 759 A1**
(43) Veröffentlichungstag der Anmeldung: **07.04.1999**
(21) Anmeldenummer: 97117124.4
(22) Anmeldetag: 02.10.1997
(51) Int. Cl.: A61K 31/66, C07F 9/09

(54) **Neue Osteoblastenspezifische Mitogene: Verfahren zu deren Herstellung und diese Verbindungen enthaltende Arzneimittel**

(71) Anmelder: Roche Diagnostics GmbH, 68298 Mannheim (DE)
(72) Erfinder: Esswein, Angelika, Dr., Dipl.-Chemikerin, 64572 Büttelborn (DE); Kling, Lothar, Dr., Humanbiologe, 68167 Mannheim (DE)

(57) **Zusammenfassung**

Gegenstand der Erfindung sind Verbindungen der Formel (I) 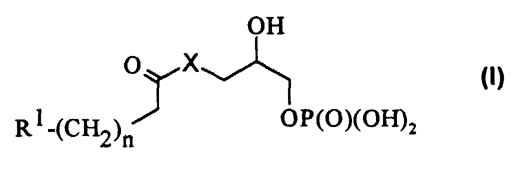 in der
R¹ = Alkyl, Alkenyl oder Alkinyl mit 6 bis 24 Kohlenstoffatomen,
n = 0 - 12
X = Sauerstoff oder NH
bedeuten sowie deren pharmakologisch unbedenkliche Salze, Ester, optisch aktive Formen, Racemate sowie Derivate, die *in vivo* zu Verbindungen der allgemeinen Fromel I metabolisiert werden können, Verfahren zu ihrer Herstellung, sowie Arzneimittel, die diese Verbindungen enthalten, zur Behandlung von Knochenstoffwechselstörungen.

## Beschreibung

Die vorliegende Erfindung betrifft osteoblastenspezifische mitogene Verbindungen der Formel (I), Verfahren zu deren Herstellung sowie Arzneimittel, die diese Verbindungen enthalten.

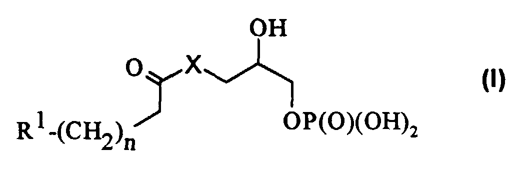

Bei gesunden Personen befinden sich die Auf- und Abbauprozesse im Knochen nahezu im Gleichgewicht, d.h. die Aktivität der Osteoblasten und Osteoklasten ist ausgeglichen. Ist dieses Gleichgewicht aber zugunsten der Osteoklasten und/oder zuungunsten der Osteoblasten gestört, kommt es zu einer Reduktion der Knochenmasse und zu einer negativen Veränderung der Knochenstruktur und -funktion.

Zur Behandlung von Knochenstoffwechselstörungen werden bisher vor allem Knochenresorptionsinhibitoren wie Oestrogene, Calcitonin und Bisphosphonate eingesetzt. Die Anwendung dieser Substanzen ist jedoch limitiert und zeigt auch nicht in allen Fällen den gewünschten Effekt. Verbindungen, die stimulierend auf den Knochenaufbau wirken und dazu beitragen eine bereits verminderte Knochenmasse zu erhöhen, sind deshalb für die Behandlung von Knochenstoffwechselstörungen von außerordentlicher Bedeutung. In den europäischen Patentanmeldungen EP-A-625522 und EP-A-524023 wurden Substanzen mit einer osteoanabolen Wirkung zur Therapie der Osteoporose beschrieben.

Es ist bekannt, daß Lysophosphatidylsäure (LPA) als intrazellulärer Lipidmessenger in verschiedenen Geweben und Zelltypen eine Rolle spielt (J. Biol. Chem. 270 (22) 12949-52, 1995; Curr. Opin. Cell. Biol. 7 (2) 203-10, 1995).

Überraschenderweise wurde nun gefunden, daß sich die Lysophosphatidylsäurederivate der vorliegenden Erfindung stimulierend auf den Knochenaufbau wirken und damit zur breiten Behandlung von Knochenstoffwechselstörungen geeignet sind. Sie lassen sich vor allem dort gut einsetzen, wo der Knochenaufbau gestört ist, d.h. sie sind besonders geeignet zur Behandlung von osteopenischen Erkrankungen des Skelettsystems wie z.B. Osteoporose, u.a. Osteogenesis imperfecta sowie zur lokalen Unterstützung der Knocherregeneration und Osteoinduktion, wie z.B. in orthopädischen und kieferheilkundlichen Indikationen, bei Frakturheilung, Osteosynthesen, Pseudoarthrosen und zur Einheilung von Knochenimplantaten.

Durch ihre Beeinflussung des Knochenstoffwechsels bilden Arzneimittel mit Lysophosphatidylsäurederivaten der vorliegenden Erfindung als Wirkstoff weiterhin eine Grundlage für die lokale und systemische Behandlung der rheumatoiden Arthritis, der Osteoarthritis und der degenerativen Arthrose.

Gegenstand der vorliegenden Erfindung sind neue Lysophosphatidylsäurederivate der allgemeinen Formel (I), 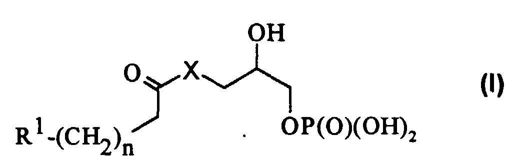 in der
R¹ = Alkyl, Alkenyl oder Alkinyl mit 6 bis 24 Kohlenstoffatomen,
n = 0 - 12
X = Sauerstoff oder NH
bedeuten, mit Ausnahme der Verbindungen (all-*cis*-5,8,11,14)-Eicosatetraensäure-2-hydroxy-3-phosphonooxy-propylester, *cis*-9,*cis*-12-Octadecadien-säure-2-hydroxy-3-phosphonooxy-propylester, (all-*cis*-9,12,15)-Ocatadecatriensäure-2-hydroxy-3-phosphonooxy-propylester oder *cis*-9-Octadecensäure-2-hydroxy-3-phosphonooxy-propylester und mit der Maßgabe, daß wenn X Sauerstoff darstellt, n in der Gruppe - (CH₂)ₙ-CH₃ nicht die Zahl 7, 9, 11, 13 oder 15 bedeutet sowie deren physiologisch verträgliche Salze, Ester, optisch aktive Formen, Racemate sowie Derivate, die *in vivo* zu Verbindungen der allgemeinen Formel (I) metabolisiert werden können, sowie die Verwendung dieser Verbindungen zur Herstellung von Arzneimitteln.

Verfahren zur Synthese der Verbindung mit X = Sauerstoff, und -(CH₂)ₙ-CH₃ mit n = 13 sind bekannt (z.B. Chem. Ber. 71 1075 (1938), Hoppe-Seyler's Z. Physiol. Chem. 347 94-101 (1966)). Verfahren zur Synthese der Verbindung mit X = Sauerstoff, und - (CH₂)ₙ-CH₃ mit n = 15 sind bekannt (z.B. Chem Phys. Lipids 1 317 (1966/67)). Verfahren zur Synthese der Verbindung mit X = Sauerstoff, und -(CH₂)ₙ-CH₃ mit n = 7 sind bekannt (z.B. Chem Phys. Lipids 18 316 (1977)).

Für die Verbindungen (all-*cis*-5,8,11,14)-*E*icosatetraensäure-2-hydroxy-3-phosphonooxy-propylester, *cis*-9,*cis*-12-*O*ctadecadiensäure-2-hydroxy-3-phosphonooxy-propylester und (all-*cis*-9,12,15)-*O*ctadecatriensäure-2-hydroxy-3-phosphonooxy-propylester ist eine Wirkung auf die Kontraktion des isolierten Rattencolons beschrieben (J. Pharm. Pharamacol. 43 774-78 (1991). Eine Wirkung auf den Blutdruck wurde für Verbindungen mit X = Sauerstoff, und -(CH₂)ₙ-CH₃ mit n = 9, 11, 13, 15, sowie für *cis*-9-*O*ctadecensäure-2-hydroxy-3 phosphonooxy-propylester, *cis*-9,*cis*-12-*O*ctadecadiensäure-2-hydroxy-3-phosphonooxy-propylester und (all-*cis*-9,12,15)-*O*ctadecatriensäure-2-hydroxy-3-phosphonooxy-propylester beschrieben (Arzneim. Forsch. 35 587-92 (1985)).

Alkyl soll in allen Fällen eine geradkettige oder verzweigte C₆-C₁₈-Alkylgruppe, wie z.B. Hexyl, Isohexyl, 2,2-Dimethylhexyl, 5-Methylhexyl, Heptyl, Isoheptyl, 6-Methylheptyl, Octyl, Isooctyl, Nonyl, Isononyl, Decyl, Isodecyl, Undecyl, Isoundecyl, Dodecyl, Isododecyl, Tridecyl, Isotridecyl, Tetradecyl, Isotetradecyl, Pentadecyl, Isopentadecyl, Hexadecyl, Heptadecyl, Isoheptadecyl oder Octadecyl, insbesondere Heptyl, Decyl und Dodecyl darstellen.

Alkenyl bedeutet in allen Fällen ein einfach oder mehrfach, gegebenfalls substituierter, ungesättigter Rest mit 6 - 20 Kohlenstoffatomen, wie z.B. Δ¹-Hexenyl, Δ¹-Octenyl, Δ⁹-Nonenyl, Δ¹-Decenyl, Δ¹⁰-Decenyl, Δ^{1,4}-Decadienyl, Δ^{1,4,7}-Decatrienyl, Δ^{1,4,7,10}-Hexadeca-tetraenyl, Δ¹-Dodecenyl, Δ⁵-Dodecenyl, Δ^{1,4}-Undecadienyl, Δ¹⁴-Tetradecenyl, insbesondere Δ¹-Decenyl, Δ^{1,4}-Decadienyl, Δ^{1,4,7}-Decatrienyl, wobei die Doppelbindungen cis oder trans, und bei mehrfach ungesättigten Verbindungen alle Kombinationen möglich sein können.

Alkinyl bedeutet in allen Fällen ein einfach oder mehrfach, gegebenenfalls substituierter, ungesättigter Rest mit 6 - 20 Kohlenstoffatomen, wie z.B. Δ¹-Decinyl, Δ¹-Noninyl, Δ^{1,3}-Tetradecadiinyl, Δ^{1,3}-Hexadecadiinyl, Δ^{1,3}-Octadecadiinyl, insbesondere Δ¹-Decinyl.

Insbesonders bevorzugt sind Verbindungen in denen X NH bedeutet.

Beispiele von physiologisch verwendbaren Salzen der Verbindung der Formel (I) sind Salze mit physiologisch verträglichen Mineralsäuren, wie Salzsäure, Schwefelsäure, schweflige Säure oder Phosphorsäure, oder mit organischen Säuren, wie Methansulfonsäure, p-Toluolsulfonsäure, Essigsäure, Trifluoressigsäure, Zitronensäure, Fumarsäure, Maleinsäure, Weinsäure, Bernsteinsäure oder Salicylsäure. Die Verbindungen der Formel (I) mit freier Carboxygruppe können auch Salze mit physiologisch verträglichen Basen bilden. Beispiele solcher Salze sind Alkalimetall-, Erdalkalimetall-, Ammonoium- und Alkylammoniumsalze, wie das Natrium-, Kalium, Calcium- oder Tetramethylammoniumsalz.

Verbindungen der allgemeinen Formel (I) enthalten mindestens ein asymmetrisches Kohlenstoffatom, daher sind auch optisch aktive Verbindungen der allgemeinen Formel (I) Gegenstand der vorliegenden Anmeldung.

Zu reinen Enantiomeren der Verbindungen der Formel (I) mit X = Sauerstoff gelangt man durch den Einsatz von optisch aktiven Alkoholen, die käuflich sind, oder die sich nach bekannten Verfahren, z.B. durch klassische Racematspaltung über Salzbildung mit optisch aktiven Säuren herstellen lassen.

Zu reinen Enantiomeren der Verbindungen der Formel (I) mit X = NH gelangt man durch den Einsatz von optisch aktiven Aminoalkoholen, die käuflich sind, oder die sich nach bekannten Verfahren, z.B. durch klassische Racematspaltung über Salzbildung mit optisch aktiven Säuren oder durch Reduktion optisch aktiver Aminosäuren herstellen lassen.

Verbindungen der allgemeinen Formel (I) mit X = Sauerstoff werden nach an sich bekannten Verfahren durch Abspalten der Schutzgruppe R₂ aus Verbindungen der allgemeinen Formel (II) hergestellt, 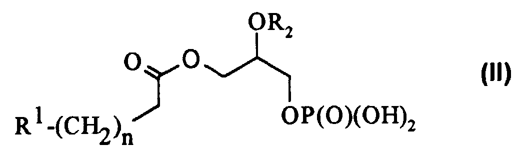 wobei R₂ eine für Hydroxylgruppen übliche Schutzgruppe darstellt.

Verbindungen der allgemeinen Formel (II) werden nach an sich bekannten Verfahren hergestellt, vorzugsweise dadurch, daß man Alkohole der allgemeinen Formel (III), 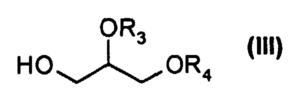 in der R₃ und R₄ für Hydroxylgruppen übliche Schutzgruppen darstellen, wobei Schutzgruppen für 1,2 Diole, wie z.B. cyclische Acetale und Ketale, bevorzugt sind,
und Carbonsäurederivaten der allgemeinen Formel (IV), 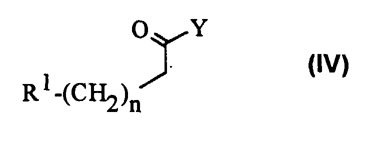 in der R₁ und n die oben angegebenen Bedeutungen haben, und Y eine Hydroxy- oder eine Aktivierungsgruppe sein kann, wobei falls Y Hydroxy bedeutet, die Aktivierung der Carboxylgruppe nach dem Carbodiimidverfahren durchgeführt werden kann, und falls Y eine Aktivierungsgruppe bedeutet, hierfür gemischte Anhydride, insbesondere mit Kohlensäureniederalkylestern wie Ethyl- oder Isobutylestern, oder aktive Ester, insbesondere p-Nitrophenyl-, 2.4.5-Trichlorphenyl-, N-Hydroxysuccinimid oder 1-Hydroxybenzotriazolester in Betracht kommen,
zu Verbindungen der allgemeinen Formel (V) 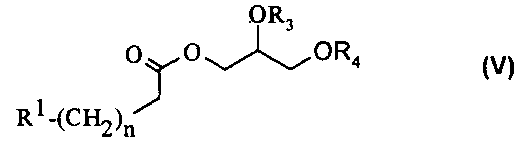 umsetzt.

Verbindungen der allgemeinen Formel (III) werden nach an sich bekannten Verfahren, vorzugsweise durch die Einführung von Schutzgruppen in Glycerin hergestellt oder sind käuflich.

Verbindungen der allgemeinen Formel (IV) werden nach bekannten Verfahren aus Verbindungen der allgemeinen Formel (VI) 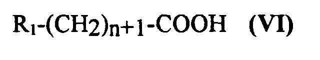 in der R₁ und n die oben angegebenen Bedeutungen haben, hergestellt.

Verbindungen der allgemeinen Formel (VI) werden nach bekannten Verfahren zur Kettenverlängerung bzw. Synthese von Carbonsäuren hergestellt oder sind käuflich.

Verbindungen der allgemeinen Formel (V) werden durch Abspalten der Schutzgruppen R₃ und R₄ in Verbindung (VII) 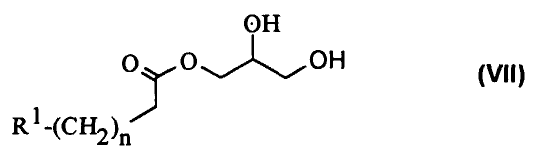 überführt.

In Verbindungen der allgemeinen Formel (VII) werden die beiden Hydroxylgruppen orthogonal durch Einführen von üblichen Hydroxylschutzgruppen R₂ und R₆ in Verbindungen der allgemeinen Formel (VIII), 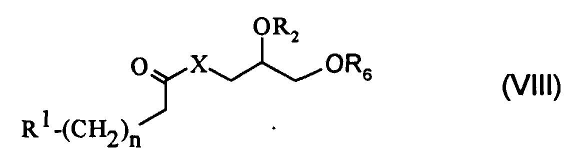 vorzugsweise durch Einführen einer Tritylgruppe (R₆) an der primären Hydroxylfunktion und einer Benzoat- oder Silylschutzgruppegruppe, wie z.B. tert.-Butyldiphenylsilyl (R₂) an der sekundären Hydroxylgruppe, überführt.

Verbindungen der allgemeinen Formel (VIII) werden nach an sich bekannten Verfahren zunächst selektiv endständig entschützt und dann durch Umsetzung mit Phosphoroxychlorid in Verbindungen der allgemeinen Formel (II) überführt.

Verbindungen der allgemeinen Formel (I) mit X = NH werden nach an sich bekannten Verfahren durch Abspalten der Schutzgruppe R₂ aus Verbindungen der allgemeinen Formel (IX) hergestellt, 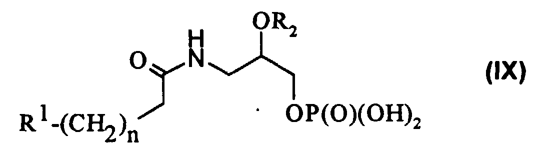 wobei R₂ eine für Hydroxylgruppen übliche Schutzgruppe darstellt.

Verbindungen der allgemeinen Formel (IX) werden nach an sich bekannten Verfahren hergestellt, vorzugsweise dadurch, daß man Amine der allgemeinen Formel (X), 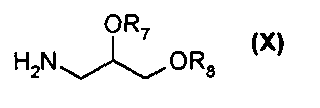 in der R₇ bzw. R₈ unabhängig voneinander Wasserstoff oder eine für Hydroxylgruppen übliche Schutzgruppe darstellen können, wobei Schutzgruppen für 1,2 Diole, wie z.B. cyclische Acetale und Ketale, bevorzugt sind,
und Carbonsäurederivaten der allgemeinen Formel (IV), 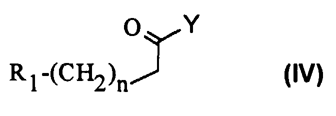 in der R₁, n und Y die oben angegebenen Bedeutungen haben
zu Verbindungen der allgemeinen Formel (XI) 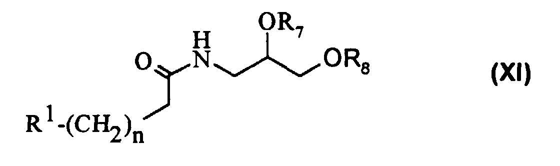 umsetzt.

Verbindungen der allgemeinen Formel (XI) erhält man für den Fall, daß R₇ und R₈ Wasserstoff bedeuten, indem analog zu dem oben beschriebenen Verfahren für die weitere Umsetzung von Verbindungen der allgemeinen Formel (VII) mit X = Sauerstoff vorgegangen wird.

Für den Fall, daß R₇ und R₈ keine orthogonalen Schutzgruppen sind, werden zunächst die Schutzgruppen nach den üblichen Methoden abgespalten und dann wie für R₇ und R₈ = Wasserstoff verfahren.

Für den Fall, daß R₇ und R₈ orthogonale Schutzgruppen sind, wird zunächst die primäre Hydroxylgruppe selektiv entschützt und dann mit Phosphoroxychlorid zu Verbindungen der allgemeinen Formel (IX) mit R₂ = R₇ umgesetzt.

Statt Phosphoroxychlorid können bei der Darstellung von Verbindungen der allgemeinen Formel (I) auch entsprechend geschützte Chlorphosphate der allgemeinen Formel (XII) 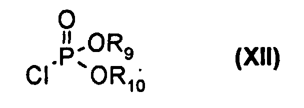 wobei für R₉ und R₁₀ übliche Schutzgruppen, insbesondere Methyl, Ethyl oder Aryl verwendet werden. Verbindungen der allgemeinen Formel (XII) sind käuflich oder nach literaturbekannten Verfahren herzustellen (Houben-Weyl)
mit Verbindungen der allgemeinen Formel (XIII) 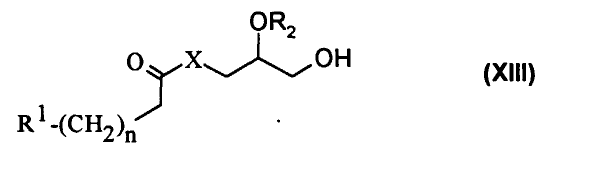 umgesetzt werden,
wobei R₁₁ eine Hydroxylschutzgruppe, vorzugsweise eine Benzoat- oder eine Silylschutzgruppe, darstellt.

Durch Abspalten der Schutzgruppen R₉, R₁₀ und R₁₁ nach den bekannten Verfahren erhält man Verbindungen der allgemeinen Formel (I).

Als Schutzguppen R₂, R₃, R₄, R₅, R₆, R₇, R₈, R₉, und R₁₀ kommen prinzipiell alle bekannten, für Hydroxylgruppen geeignete, Schutzgruppen in Frage, wie sie auch in Th. Greene, P. Wuts " Protective Groups in Organic Synthesis" 2. Aufl. 1991 J. Wiley&Sons beschrieben sind. Die Einführung und Abspaltung erfolgt nach den dort beschriebenen üblichen Vefahren.

Verbindungen der Formel (I) können in flüssiger,fester oder in Form von Aerosolen oral, enteral, parenteral, topisch, nasal, pulmonal oder rectal in allen üblichen nichttoxischen pharmazeutisch akzeptierten Trägermaterialien, Adjuvantien und Zusätzen verabbreicht werden. Die Verbindungen der Formel (I) können auch lokal an/in den Knochen (evtl. unter chirurgischem Eingriff) appliziert werden. Der Begriff parenteral umfaßt dabei subcutane , intravenöse und intramuskuläre Zufuhr oder Infusionen. Orale Applikationsformen können z.B. Tabletten, Kapseln, Dragees, Sirupe, Lösungen, Suspensionen, Emulsionen, Elixiere etc. sein, die einen oder mehrere Zusätze aus den folgenden Gruppen enthalten können, wie z.B. Geschmacksstoffe, Süßstoffe, Farbstoffe und Konservierungsmittel. Orale Applikationsformen enthalten den wirksamen Bestandteil zusammen mit nichttoxischen, pharmazeutisch akzeptierten Trägermaterialien, die zur Herstellung von Tabletten, Kapseln, Dragees usw. geeignet sind, wie z.B. Calciumcarbonat, Natriumcarbonat, Lactose, Calciumphosphat oder Natriumphosphat; Stärke, Mannit, Methylcellulose, Talkum, hochdisperse Kieselsäuren, höhermolekulare Fettsäuren (wie Stearinsäure), Erdnußöl, Olivenöl, Paraffin, Miglyol, Gelatine, Agar-Agar, Magnesiumstearat, Bienenwachs, Cetylalkohol, Lecithin, Glycerol, tierische und pflanzliche Fette, feste hochmolekulare Polymere (wie Polyethylenglykole). Tabletten, Kapseln, Dragees usw. können mit einem entsprechenden Überzug, wie z.B. Glycerylmonostearat oder Glyceryldistearat versehen werden, so daß unerwünschte Nebenwirkungen im Magen verhindert werden, oder es durch die verzögerte Absorption im Gastrointestinaltrakt zu einer längeren Wirkungsdauer kommt. Als Injektionsmedium kommen vorzugsweise sterile injizierbare wäßrige oder ölige Lösungen oder Suspensionen zur Anwendung, welche die üblichen Zusätze, wie Stabilisierungsmittel und Lösungsvermittler enthalten. Derartige Zusätze können z.B. Wasser, isotonische Kochsalzlösung, 1,3-Butandiol, Fettsäuren (wie Ölsäure), Mono- und Diglyceride, oder Miglyol sein. Für die rectale Anwendung können alle geeigneten nicht irritierenden Zusätze verwendet werden, die bei normalen Temperaturen fest und bei Rectaltemperatur flüssig sind, wie z. B. Kakaobutter und Polyethylenglykol. Für die Anwendung als Aerosol kommen die pharmazeutisch üblichen Trägermedien zur Anwendung. Für den äußerlichen Gebrauch finden Cremes, Tinkturen, Gele, Lösungen oder Suspensionen usw. mit den pharmazeutisch üblichen Zusätzen Anwendung.

Die Dosierung kann von verschiedenen Faktoren, wie Applikationsweise, Spezies, Alter und/oder individuellem Zustand abhängen. Die täglichen oder in Intervallen zu verabreichenden Dosen liegen bei 1 - 1000 mg/Mensch, vorzugsweise bei 10 - 250 mg/Mensch und können auf einmal oder mehrere Male verteilt eingenommen werden.

Die Verbindungen der Formel (I) können lokal an/in den Knochen (evtl. unter chirurgischem Eingriff) appliziert werden. Die Applikation direkt an/in den Knochen (evtl. unter chirurgischem Eingriff) kann entweder in Lösung oder Suspension zweckmäßig durch Infusion oder Injektion lokal oder trägergebunden erfolgen. Trägergebundene Verbindungen der Formel (I) können beispielsweise als Gele, Pasten, Feststoff oder als Beschichtung auf Implantaten appliziert werden.

Als Träger werden biokompatible und vorzugsweise bioabbaubare Materialien verwendet. Vorzugsweise induzieren die Materialien selbst zusätzlich noch die Wundheilung oder die Osteogenese.

Zur lokalen Applikation ist es bevorzugt, die Verbindungen der Formel (I) in polymere Gele oder Filme einzubetten, dadurch zu immobilisieren und diese Präparation direkt auf die zu behandelnde Stelle am Knochen aufzutragen. Derartige polymere Basisgele oder Filme bestehen beispielsweise aus Glycerin, Methylcellulose, Hyaluronsäure, Polyethylenoxiden und/oder Polyoxameren. Ebenfalls geeignet sind Kollagen, Gelatine und Alginate und sind beispielsweise in WO 93/00050 und WO 93/20859 beschrieben. Weitere Polymere sind Polymilchsäure (PLA) und Copolymere aus Milchsäure und Glykolsäure (PLPG) (Hollinger et al., J. Biomed. Mater. Res. 17 71-82 (1983)) sowie das Knochenderivat "Demineralized Bone Matrix" (DBM) (Guterman et al. Kollagen Rel. Res. 8 419-4319 (1988). Ebenfalls geeignet sind Polymere, wie sie zum Beispiel zur Adsorption für TGFβ verwendet werden und in der EP-A 0 616 814 und der EP-A 0 567 391 beschrieben sind und synthetische Knochenmatrices gemäß WO 91/18558.

Ebenso geeignet als Träger für die Verbindungen der Formel (I) sind Materialien, die üblicherweise bei der Implantation von Knochenersatzstoffen oder von sonstigen therapeutischen Wirkstoffen verwendet werden. Solche Träger basieren beispielsweise auch auf Calciumsulfat, Tricalciumphosphat, Hydroxyapatit und seine biodegradablen Derivate und Polyanhydriden. Außer diesen bioabbaubaren Trägern sind auch Träger geeignet, die nicht bioabbaubar sind, aber biokompatibel sind. Solche Träger sind beispielsweise gesinterter Hydroxylapatit, Bioglas, Aluminate oder andere keramische Materialien (z.B. Calcium-Aluminat-Phosphat). Diese Materialien werden bevorzugt in Kombination mit den bioabbaubaren Materialen, wie insbesondere Polymilchsäure, Hydroxylapatit, Kollagen oder Tricalciumphosphat angewendet. Weitere nicht abbaubare Polymere sind beispielsweise im US-Patent 4,164,560 beschrieben.

Besonders bevorzugt ist es, einen Träger zu verwenden, der die Verbindungen der Formel (I) kontinuierlich am Wirkort freisetzt. Hierfür besonders geeignet sind z.B. "slow release pellets" von Innovative Research of America, Toledo, Ohio, USA. Besonders bevorzugt werden Pellets verwendet, welche die Verbindungen der Formel (I) über mehrere Tage, vorzugsweise bis zu 100 Tagen bei einer täglichen Dosis von 1-10 mg/kg pro Tag, freisetzen.

Die Dosierung kann von verschiedenen Faktoren, wie Applikationsweise, Spezies, alter und/oder individuellem Zustand abhängen. Die täglich zu verabreichende Dosis wirksamer Substanz liegt bei 0.01 mg bis ungefähr 100 mg/kg Körpergewicht, vorzugsweise bei 0.1 bis 10 mg/kg Körpergewicht und kann auf einmal oder auf mehrere Male verteilt appliziert werden.

Bevorzugt im Sinne der vorliegenden Erfindung sind außer in den Beispielen genannten Verbindungen und durch Kombination aller in den Ansprüchen genannten bedeutungen der Substitutenten ableitbaren Verbindungen, die folgenden Lysophosphatidylsäurederivate, sowie deren Natrium- und Kaliumsalze:

### Bevorzugte Verbindungen (BV):

(1) Octansäure-2-hydroxy-3-phosphonooxy-propylester
(2) 7-Methyloctansäure-2-hydroxy-3-phosphonooxy-propylester
(3) 7,7-*D*imethyloctansäure-2-hydroxy-3-phosphonooxy-propylester
(4) Nonansäure-2-hydroxy-3-phosphonooxy-propylester
(5) 4-Methylnonansäure-2-hydroxy-3-phosphonooxy-propylester
(6) 8-Methylnonansäure-2-hydroxy-3-phosphonooxy-propylester
(7) Undecansäure-2-hydroxy-3-phosphonooxy-propylester
(8) 10-Methylundecansäure-2-hydroxy-3-phosphonooxy-propylester
(9) 11-Methyldodecansäure-2-hydroxy-3-phosphonooxy-propylester
(10) Tridecansäure-2-hydroxy-3-phosphonooxy-propylester
(11) 12-Methyltridecansäure-2-hydroxy-3-phosphonooxy-propylester
(12) 13-Methyltetradecansäure-2-hydroxy-3-phosphonooxy-propylester
(13) Pentadecansäure-2-hydroxy-3-phosphonooxy-propylester
(14) 14-Methylpentadecansäure-2-hydroxy-3-phosphonooxy-propylester
(15) 15-Methylhexadecansäure-2-hydroxy-3-phosphonooxy-propylester
(16) Heptadecansäure-2-hydroxy-3-phosphonooxy-propylester
(17) 16-Methylheptadecansäure-2-hydroxy-3-phosphonooxy-propylester
(18) 17-Methyloctadecansäure-2-hydroxy-3-phosphonooxy-propylester
(19) Nonadecansäure-2-hydroxy-3-phosphonooxy-propylester
(20) 18-Methylnonadecansäure-2-hydroxy-3-phosphonooxy-propylester
(21) Eicosansäure-2-hydroxy-3-phosphonooxy-propylester
(22) 19-Methyleicosansäure-2-hydroxy-3-phosphonooxy-propylester
(23) 19-Methyleicosansäure-2-hydroxy-3-phosphonooxy-propylester
(24) Heneicosansäure-2-hydroxy-3-phosphonooxy-propylester
(25) Docosansäure-2-hydroxy-3-phosphonooxy-propylester
(26) Tricosansäure-2-hydroxy-3-phosphonooxy-propylester
(27) Tetracosansäure-2-hydroxy-3-phosphonooxy-propylester
(28) Heptacosansäure-2-hydroxy-3-phosphonooxy-propylester
(29) Octacosansäure-2-hydroxy-3-phosphonooxy-propylester
(30) Triacontansäure-2-hydroxy-3-phosphonooxy-propylester
(31) 6-Heptensäure-2-hydroxy-3-phosphonooxy-propylester
(32) *Trans*-9-hexadecensäure-2-hydroxy-3-phosphonooxy-propylester
(33) (all-*cis*-11,14,17)-*E*icosatriensäure-2-hydroxy-3-phosphonooxy-propylester
(34) *cis*-10-Heptadecensäure-2-hydroxy-3-phosphonooxy-propylester
(35) *cis*-10-Nonadecensäure-2-hydroxy-3-phosphonooxy-propylester
(36) *cis*-3,*cis*-6-Nonadiensäure-2-hydroxy-3-phosphonooxy-propylester
(37) *cis*-10-Pentadecensäure-2-hydroxy-3-phosphonooxy-propylester
(38) *cis*-12-*O*ctadecensäure-2-hydroxy-3-phosphonooxy-propylester
(39) *cis*-13-*O*ctadecensäure-2-hydroxy-3-phosphonooxy-propylester
(40) *cis*-7-*O*ctadecensäure-2-hydroxy-3-phosphonooxy-propylester
(41) *cis*-8-*E*icosensäure-2-hydroxy-3-phosphonooxy-propylester
(42) *trans*-9-Tetradecensäure-2-hydroxy-3-phosphonooxy-propylester
(43) *trans*-9-*O*ctadecensäure-2-hydroxy-3-phosphonooxy-propylester
(44) (all-*trans*-9,11,13,15)-*O*ctadecatetraensäure-2-hydroxy-3-phosphonooxy-propylester
(45) (all-*cis*-9,11,13,15)-*O*ctadecatetraensäure-2-hydroxy-3-phosphonooxy-propylester
(46) *cis*-11-*O*ctadecensäure-2-hydroxy-3-phosphonooxy-propylester
(47) (all-*cis*-13,16,19)-*D*ocosatriensäure-2-hydroxy-3-phosphonooxy-propylester
(48) (all-*cis*-13,16,19)-*D*ocosatriensäure-2-hydroxy-3-phosphonooxy-propylester
(49) (all-*cis*-8,11,14)-*E*icosatriensäure-2-hydroxy-3-phosphonooxy-propylester
(50) *trans*-11-Octadecensäure-2-hydroxy-3-phosphonooxy-propylester
(51) *trans*-13-Docosensäure-2-hydroxy-3-phosphonooxy-propylester
(52) *trans*-9,*trans*-12-*O*ctadecadiensäure-2-hydroxy-3-phosphonooxy-propylester
(53) *cis*-9-Tetradecensäure-2-hydroxy-3-phosphonooxy-propylester
(54) *cis*-9-Hexadecensäure-2-hydroxy-3-phosphonooxy-propylester
(55) 10-Undecensäure-2-hydroxy-3-phosphonooxy-propylester
(56) *cis*-11,*cis*-14-*E*icosadiensäure-2-hydroxy-3-phosphonooxy-propylester
(57) *cis*-11-*E*icosensäure-2-hydroxy-3-phosphonooxy-propylester
(58) *cis*-15-Tetracosensäure-2-hydroxy-3-phosphonooxy-propylester
(59) 11-*D*odecensäure-2-hydroxy-3-phosphonooxy-propylester
(60) 9-*D*ecensäure-2-hydroxy-3-phosphonooxy-propylester
(61) 16-Heptadecensäure-2-hydroxy-3-phosphonooxy-propylester
(62) (all-*cis*-11,14,17)-*E*icosatriensäure-2-hydroxy-3-phosphonooxy-propylester
(63) *cis*-13-*E*icosensäure-2-hydroxy-3-phosphonooxy-propylester
(64) (all-*cis*-7,10,13,16)-*D*ocosatetraensäure-2-hydroxy-3-phosphonooxy-propylester
(65) 22-Tricosensäure-2-hydroxy-3-phosphonooxy-propylester
(66) 9-Tetradecinsäure-2-hydroxy-3-phosphonooxy-propylester
(67) 13-*E*icosinsäure-2-hydroxy-3-phosphonooxy-propylester
(68) 10,12-Nonacosadiinsäure-2-hydroxy-3-phosphonooxy-propylester
(69) 10,12-*O*ctadecadiinsäure-2-hydroxy-3-phosphonooxy-propylester
(70) 9-*O*ctadecinsäure-2-hydroxy-3-phosphonooxy-propylester
(71) 10-Undecinsäure-2-hydroxy-3-phosphonooxy-propylester
(72) 10,12-Tricosadiinsäure-2-hydroxy-3-phosphonooxy-propylester
(73) 10,12-Pentacosadiinsäure-2-hydroxy-3-phosphonooxy-propylester
(74) 10,12-Heptacosadiinsäure-2-hydroxy-3-phosphonooxy-propylester
(75) Octansäure-2-hydroxy-3-phosphonooxy-propylamid
(76) 7-Methyloctansäure-2-hydroxy-3-phosphonooxy-propylamid
(77) 7,7-*D*imethyloctansäure-2-hydroxy-3-phosphonooxy-propylamid
(78) Nonansäure-2-hydroxy-3-phosphonooxy-propylamid
(79) 4-Methylnonansäure-2-hydroxy-3-phosphonooxy-propylamid
(80) 8-Methylnonansäure-2-hydroxy-3-phosphonooxy-propylamid
(81) Decansäure-2-hydroxy-3-phosphonooxy-propylamid
(82) Undecansäure-2-hydroxy-3-phosphonooxy-propylamid
(83) 10-Methylundecansäure-2-hydroxy-3-phosphonooxy-propylamid
(84) Dodecansäure-2-hydroxy-3-phosphonooxy-propylamid
(85) 11-Methyldodecansäure-2-hydroxy-3-phosphonooxy-propylamid
(86) Tridecansäure-2-hydroxy-3-phosphonooxy-propylamid
(87) 12-Methyltridecansäure-2-hydroxy-3-phosphonooxy-propylamid
(88) Tetradecansäure-2-hydroxy-3-phosphonooxy-propylamid
(89) 13-Methyltetradecansäure-2-hydroxy-3-phosphonooxy-propylamid
(90) Pentadecansäure-2-hydroxy-3-phosphonooxy-propylamid
(91) 14-Methylpentadecansäure-2-hydroxy-3-phosphonooxy-propylamid
(92) Hexadecansäure-2-hydroxy-3-phosphonooxy-propylamid
(93) 15-Methylhexadecansäure-2-hydroxy-3-phosphonooxy-propylamid
(94) Heptadecansäure-2-hydroxy-3-phosphonooxy-propylamid
(95) 16-Methylheptadecansäure-2-hydroxy-3-phosphonooxy-propylamid
(96) Octadecansäure-2-hydroxy-3-phosphonooxy-propylamid
(97) 17-Methyloctadecansäure-2-hydroxy-3-phosphonooxy-propylamid
(98) Nonadecansäure-2-hydroxy-3-phosphonooxy-propylamid
(99) 18-Methylnonadecansäure-2-hydroxy-3-phosphonooxy-propylamid
(100) Eicosansäure-2-hydroxy-3-phosphonooxy-propylamid
(101) 19-Methyleicosansäure-2-hydroxy-3-phosphonooxy-propylamid
(102) 19-Methyleicosansäure-2-hydroxy-3-phosphonooxy-propylamid
(103) Heneicosansäure-2-hydroxy-3-phosphonooxy-propylamid
(104) Docosansäure-2-hydroxy-3-phosphonooxy-propylamid
(105) Tricosansäure-2-hydroxy-3-phosphonooxy-propylamid
(106) Tetracosansäure-2-hydroxy-3-phosphonooxy-propylamid
(107) Heptacosansäure-2-hydroxy-3-phosphonooxy-propylamid
(108) Octacosansäure-2-hydroxy-3-phosphonooxy-propylamid
(109) Triacontansäure-2-hydroxy-3-phosphonooxy-propylamid
(110) 6-Heptensäure-2-hydroxy-3-phosphonooxy-propylamid
(111) *Trans*-9-hexadecensäure-2-hydroxy-3-phosphonooxy-propylamid
(112) (all-*cis*-11,14,17)-*E*icosatriensäure-2-hydroxy-3-phosphonooxy-propylamid
(113) (all-*cis*-5,8,11,14)-*E*icosatetraensäure-2-hydroxy-3-phosphonooxy-propylamid
(114) *cis*-10-Heptadecensäure-2-hydroxy-3-phosphonooxy-propylamid
(115) *cis*-10-Nonadecensäure-2-hydroxy-3-phosphonooxy-propylamid
(116) *cis*-3,*cis*-6-Nonadiensäure-2-hydroxy-3-phosphonooxy-propylamid
(117) *cis*-10-Pentadecensäure-2-hydroxy-3-phosphonooxy-propylamid
(118) *cis*-12-Octadecensäure-2-hydroxy-3-phosphonooxy-propylamid
(119) *cis*-13-*O*ctadecensäure-2-hydroxy-3-phosphonooxy-propylamid
(120) *cis*-7-*O*ctadecensäure-2-hydroxy-3-phosphonooxy-propylamid
(121) *cis*-8-*E*icosensäure-2-hydroxy-3-phosphonooxy-propylamid
(122) *trans*-9-Tetradecensäure-2-hydroxy-3-phosphonooxy-propylamid
(123) *cis*-9,*cis*-12-*O*ctadecadiensäure-2-hydroxy-3-phosphonooxy-propylamid
(124) *trans*-9-*O*ctadecensäure-2-hydroxy-3-phosphonooxy-propylamid
(125) *cis*-9-*O*ctadecensäure-2-hydroxy-3-phosphonooxy-propylamid
(126) (all-*trans*-9,11,13,15)-*O*ctadecatetraensäure-2-hydroxy-3-phosphonooxy-propylamid
(127) (all-*cis*-9,11,13,15)-*O*ctadecatetraensäure-2-hydroxy-3-phosphonooxypropylamid
(128) *cis*-11-*O*ctadecensäure-2-hydroxy-3-phosphonooxy-propylamid
(129) (all-*cis*-13,16,19)-*D*ocosatriensäure-2-hydroxy-3-phosphonooxy-propylamid
(130) (all-cis-13,16,19)-*D*ocosatriensäure-2-hydroxy-3-phosphonooxy-propylamid
(131) (all-*cis*-9,12,15)-*O*ctadecatriensäure-2-hydroxy-3-phosphonooxy-propylamid
(132) (all-*cis*-8,11,14)-*E*icosatriensäure-2-hydroxy-3-phosphonooxy-propylamid
(133) *trans*-11-*O*ctadecensäure-2-hydroxy-3-phosphonooxy-propylamid
(134) *trans*-13-Docosensäure-2-hydroxy-3-phosphonooxy-propylamid
(135) *trans*-9,*trans*-12-Octadecadiensäure-2-hydroxy-3-phosphonooxy-propylamid
(136) *cis*-9-Tetradecensäure-2-hydroxy-3-phosphonooxy-propylamid
(137) *cis*-9-Hexadecensäure-2-hydroxy-3-phosphonooxy-propylamid
(138) 10-Undecensäure-2-hydroxy-3-phosphonooxy-propylamid
(139) *cis*-11,*cis*-14-*E*icosadiensäure-2-hydroxy-3-phosphonooxy-propylamid
(140) *cis*-11-*E*icosensäure-2-hydroxy-3-phosphonooxy-propylamid
(141) *cis*-15-Tetracosensäure-2-hydroxy-3-phosphonooxy-propylamid
(142) 11-*D*odecensäure-2-hydroxy-3-phosphonooxy-propylamid
(143) 9-*D*ecensäure-2-hydroxy-3-phosphonooxy-propylamid
(144) 16-Heptadecensäure-2-hydroxy-3-phosphonooxy-propylamid
(145) (all-*cis*-11,14,17)-*E*icosatriensäure-2-hydroxy-3-phosphonooxy-propylamid
(146) *cis*-13-*E*icosensäure-2-hydroxy-3-phosphonooxy-propylamid
(147) *cis*-13,*cis*-13-*D*ocosadiensäure-2-hydroxy-3-phosphonooxy-propylamid
(148) (all-*cis*-7,10,13,16)-*D*ocosatetraensäure-2-hydroxy-3-phosphonooxy-propylamid
(149) 22-Tricosensäure-2-hydroxy-3-phosphonooxy-propylamid
(150) 9-Tetradecinsäure-2-hydroxy-3-phosphonooxy-propylamid
(151) 13-*E*icosinsäure-2-hydroxy-3-phosphonooxy-propylamid
(152) 10,12-Nonacosadiinsäure-2-hydroxy-3-phosphonooxy-propylamid
(153) 10,12-*O*ctadecadiinsäure-2-hydroxy-3-phosphonooxy-propylamid
(154) 9-*O*ctadecinsäure-2-hydroxy-3-phosphonooxy-propylamid
(155) 10-Undecinsäure-2-hydroxy-3-phosphonooxy-propylamid
(156) 10,12-Tricosadiinsäure-2-hydroxy-3-phosphonooxy-propylamid
(157) 10,12-Pentacosadiinsäure-2-hydroxy-3-phosphonooxy-propylamid
(158) 10,12-Heptacosadiinsäure-2-hydroxy-3-phosphonooxy-propylamid

Die nachfolgenden Beispiele zeigen einige Verfahrensvarianten, die zur Synthese der erfindungsgemäßen Verbindungen verwendet werden können. Sie sollten jedoch nicht eine Einschränkung des Erfindungsgegenstandes darstellen. Die Struktur der Verbindungen wurde durch ¹H-, ³¹P- und gegebenenfalls durch ¹³C-NMR-Spektroskopie gesichert. Die Reinheit der Substanzen wurde mittels C, H, N, P Analyse, sowie dünnschichtchromatographisch bestimmt.

### Beispiel 1

### cis-9-Octadecensäure-2-hydroxy-3-phosphonooxy-propylester

### cis-9-Octadecensäure-2,3-O-isopropyliden-propylester (1)

Zu einer Lösung aus 15.4 g (116 mmol) 2,2-Dimethyl-4-hydroxy-methyldioxolan in 100 ml Pyridin wird bei RT 41.2g (116 mmol) 85% iges Oelsäurechlorid zugetropft. Nach 12 h bei RT wird das Pyridin abgezogen, der Rückstand 2 x mit 50 ml Toluol versetzt und eingedampft. Der Rückstand wird in 200 ml Diethylether aufgenommen und 2 x mit 50 ml 1 N HCl und 50 ml gesättigter NaCl extrahiert. Die Etherphase wird mit Mg₂SO₄ getrocknet, abfiltriert und eingeengt. Der Rückstand wird über eine Kieselgel-Flashsäule mit Heptan/Essigsäureethylester (9:1) gereinigt. Ausbeute 26.3 g (61%).

### cis-9-Octadecensäure-2,3-di-hydroxy-propylester (2)

Eine Lösung aus 24 g (60.5 mmol) 1 werden in einem Gemisch aus THF/Wasser (6:1) gelöst und auf 0° C abgekühlt. Dann wird Trifluoressigsäure (24 ml) zugetropft und die Mischung wird für weitere 3 h bei 0°C gerührt. Es wird auf RT erwärmt. Nach 12 h wird auf 0°C abgekühlt und mit konz. Ammoniak neutralisiert. Das THF wird abdestiliiert und der Rückstand mit Diethylether extrahiert. Die vereinigten org. Phasen werden über Mg₂SO₄ getrocknet, abfiltriert und eingeengt. Der Rückstand wird über eine Kieselgelsäule mit Heptan/Essigsäureethylester (1:1) gereinigt. Ausbeute: 68% farbloses Oel.

### cis-9-Octadecensäure-2-hydroxy-3-triphenylmethoxy-propylester (3)

Zu einer Lösung aus 2 (21 mmol) in 80 ml Dichlormethan/Pyridin (1:1) wird Triphenylmethylchlorid (27 mmol) zugetropft und 48 h bei RT gerührt. Das Lösungsmittel wird abgezogen und der Rückstand 2 x mit je 50 ml Toluol versetzt und eingeengt. Der Rückstand wird mit 100 ml H₂O verdünnt und mit 3 x je 50 ml Dichlormethan extrahiert. Die vereinigten org. Phasen werden mit 50 ml kalter 5% HCl und 50 ml gesättigter NaCl gewaschen, über Mg₂SO₄ getrocknet, abfiltriert und eingeengt. Der Rückstand wird durch Chromatographie an Kieselgel mit Heptan/Essigsäureethylester (5:1) gereinigt. Ausbeute: 88 % farbloses Oel.

### cis-9-Octandecensäure-2-benzoyloxy-3-triphenylmethoxy-propylester (4)

Zu einer Lösung aus 3 (5 mmol) in 30 ml DMF wird bei 0°C Imidazol (20 mmol) zugegeben. Zu dieser Mischung wird dann tert.Butyldiphenylsilylchlorid zugetropft. Die Mischung wird langsam auf RT erwärmt. Nach 6 h bei RT wird auf Eiswasser gegeben und mit Essigsäureethylester extrahiert. Die vereinigten org. Phasen werden mit ges. NaCl-Lösung und Wasser gewaschen, über Mg₂SO₄ getrocknet, abfiltriert und eingeengt. Der Rückstand wird durch Kieselgelchromatographie mit Isohexan/Essigsäureethylester (9:1) gereinigt. Ausbeute: 95 % farbloses Oel.

### cis-9-Octadecensäure-2-tert.butyldiphenylsilylether-3-hydroxy-propylester (5)

Zu einer Lösung aus 4 (4.65 mmol) in 50 ml Dichlormethan wird bei RT 4 ml Trifluoressigsäure langsam zugetropft. Nach 3 h wird mit Wasser und mit ges. Natriumhydrogencarbonatlösung gewaschen. Die org. Phasen werden über Mg₂SO₄ getrocknet, abfiltriert und eingeengt. Der Rückstand wird durch Flashchromatographie an Kieselgel mit Isohexan/Essigsäureethylester (7:1) gereinigt. Ausbeute: 54 % farbloses Oel.

### cis-9-Octadecensäure-2-tert.butyldiphenylsilylether-3-phosphonooxy-propylester (6)

Unter Stickstoffwird eine Lösung aus Phosphoroxychlorid (3 mmol) in 5 ml Tetrahydrofuran auf 0°C abgekühlt und eine Lösung aus 5 (2.75 mmol) und Pyridin (9.3 mmol) in 15 ml Tetrahydrofuran zugetropft. Die Mischung wird 30 min bei 0°C gerührt. Dann wird mit 3 ml Wasser versetzt und 20 h bei RT gerührt. Anschließend wird durch die tropfenweise Zugabe von 1 N HCl angesäuert und mit 3 x mit je 25 ml Essigsäurediethylester extrahiert. Die vereinigten org. Phasen werden über Mg₂SO₄ getrocknet, abfiltriert und eingeengt. Der Rückstand wird über Kieselgel zunächst mit Essigsäureethylester, dann mit Methanol chromatographiert. Ausbeute: 71% farbloses Oel.

### cis-9-Octadecensäure-2-hydroxy-3-phosphonooxy-propylester

6 (1 mmol) werden in 10 ml Dichlormethan gelöst und mit 25 ml einer 1% methanolischen NaOH-Lösung versetzt. Nach 20 h bei RT wird eingeengt und der Rückstand mit 1 N HCl angesäuert. Die Mischung wird mit Essigsäureethylester extrahiert. Die vereinigten org. Phasen werden mit 20 ml H₂O gewaschen und über Mg₂SO₄ getrocknet, abfiltriert und engeengt. Der Rückstand wird mit Methanol behandelt. Ausbeute: 78 % farblose Kristalle.

### Beispiel 2

### cis-9-Octadecensäure-2-hydroxy-3-phosphonooxy-propylamid

### cis-9-Octadecensäuresuccinimid (8)

Zu einer Lösung aus Oelsäure (30 g, 106.2 mmol) in 150 ml THF wird bei 0°C Dicyclcohexylcarbodiimid ((24.1 g, 117 mmol) gelöst in THF zugegeben. Nach 20 min wird die Mischung mit N-Hydroxysuccinimid (13.5 g, 117 mmol) versetzt. Die Mischung wird langsam auf RT erwärmt. Nach 18 h bei RT wird auf 0°C abgekühlt und der Niederschlag abgesaugt. Das Filtrat wird eingeengt und über eine Kieselgelsäule mit Essigsäureethylester/Hepten (1:3) gereingt. Ausbeute: 83% farbloses Wachs.

### cis-9-Octadecensäure-2,3-dihydroxy-propylamid (9)

Zu einer Lösung aus cis-9-Octadecensäuresuccinimid (18.3 g, 48.2 mmol) in 50 ml Acetonitril werden 1-Amino-2,3-propandiol (4.4 g, 48.2 mmol) in 50 ml H₂O zugegeben. Nach 12 h rühren bei RT wird das Acetonitril abdestilliert und der Rückstand mit Essigsäureethylester extrahiert. Die vereinigten org. Phasen werden gesättigter NaCl extrahiert. Die org. Phase wird mit Mg₂SO₄ getrocknet, abfiltriert und eingeengt. Der Rückstand wird mit Isohexan kristallisiert. Ausbeute: 87 % farbloses Pulver.

### cis-9-Octadecensäure-2-hydroxy-3-triphenylmethoxy-propylamid (10)

Zu einer Lösung aus 9 (28 mmol) in 80 ml Dichlormethan/Pyridin (1:1) wird Triphenylmethylchlorid (36 mmol) zugetropft und 48 h bei RT gerührt. Das Lösungsmittel wird abgezogen und der Rückstand 2 x mit je 50 ml Toluol versetzt und eingeengt. Der Rückstand wird mit 100 ml H₂O versetzt und mit 3 x je 50 ml Dichlormethan extrahiert. Die vereinigten org. Phasen werden mit 50 ml kalter 5% HCl und 50 ml gesättigter NaCl gewaschen, über Mg₂SO₄ getrocknet, abfiltriert und eingeengt. Der Rückstand wird durch Chromatographie an Kieselgel mit Heptan/Essigsäureethylester (2:1) gereinigt. Ausbeute: 81 % farbloses Oel.

### cis-9-Octandecensäure-2-benzoyloxy-3-triphenylmethoxy-propylamid (11)

Zu einer Lösung aus 10 (27.3 mmol) in 80 ml Dichlormethan/Pyridin (1:1) wird bei 0°C Benzoylchlorid (30 mmol) zugetropft.. Man läßt langsam auf RT kommen und rührt noch 4 h bei RT nach. Dann wird die Mischung eingeengt, 2 x mit je 50 ml Toluol versetzt und eingeengt. Der Rückstand wird mit 100 ml H₂O versetzt und 3 x mit je 100 ml Dichlormethan extrahiert. Die vereinigten org. Phasen werden mit kalter 5% HCl , dann mit 50 ml gesättigter NaCl-Lösung gewaschen, über Mg₂SO₄ getrocknet, abfiltriert und eingeengt. Der Rückstand wird durch Kieselgelchromatographie mit Heptan/Essigsäureethylester (3:1) gereinigt. Ausbeute: 87% farbloses Oel.

### cis-9-Octadecensäure-2-benzoyloxy-3-hydroxy-propylamid (12)

Unter Stickstoff wird eine Lösung aus Phosphoroxychlorid (7.1 mmol) in 10 ml (22 mmol) in 30 ml Tetranydrofuran zugetropft. Die Mischung wird 30 min 0°C gerührt. Dann wird mit 5 ml Wasser versetzt und 20 h bei RT gerührt. Die Mischung wird durch die tropfenweise Zugabe von 1 N HCl angesäuert. Es wird 3 x mit je 50 ml Essigsäurediethylester extrahiert, die vereinigten org. Phasen über Mg₂SO₄ getrocknet, abfiltriert und eingeengt. Der Rückstand wird über Kieselgel zunächst mit Essigsäureethylester, dann mit Methanol chromatographiert. Ausbeute: 54% farbloses Oel.

### cis-9-Octadecensäure-2-hydroxy-3-phosphonooxy-propylamid

13 (1.76 mmol) werden in 20 ml Dichlormethan gelöst und mit 50 ml einer 1% methanolischen NaOH-Lösung versetzt. Nach 20 h bei RT wird eingeengt und der Rückstand mit 1 N HCl angesäuert. Die Mischung wird mit Essigsäureethylester extrahiert. Die vereinigten org. Phasen werden mit 50 ml H₂O gewaschen und über Mg₂SO₄ getrocknet, abfiltriert und engeengt. Der Rückstand wird mit Methanol behandelt. Ausbeute: 82 % farblose Kristalle.

### Beispiel 3

Verbindungen der Formel (I) wurden in einem DNA-Synthese-Assay in Primärkulturen von Osteoblasten aus fetalen Rattenkalvarien untersucht. Die Experimente wurden in Anlehnung an Pfeilschifter et al., Endocrinology 126, 703 (1990) durchgeführt.

### Versuchsbeschreibung: BrdU-Methode

Die DNA-Syntheseleistung als Surrogatparameter für die Proliferation wurde mittels des 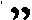 Cell Proliferation ELISA, BrdU (colorimetric)" der Firma Boehringer Mannheim, Mannheim, Deutschland, bestimmt.

Die Gewinnung von primären Osteoblasten erfolgte durch sequentielle Abdauung mittels Collagenase aus fetalen Rattenkalvarien. Dabei wurden 5 Zellfraktionen erhalten. Der Pool aus den Zellfraktionen 3-5 wurde in vitro kultiviert. Die Kultur der Zellen erfolgte in einem Inkubator bei einer relativen Luftfeuchte von 95%, einem CO₂-Gehalt von 5% und einer Temperatur von 37°C. Die Untersuchungen der Prüfsubstanzen erfolgte in Kulturen der ersten, zweiten oder dritten Zellpassage.

Für die Untersuchungen wurden die Zellen mindestens 96 Stunden vor Aufgabe der Prüfsubstanzen in einer Zellzahl von 7x10³ Zellen (in 100 µl Kulturmedium) / Well in Mikrotiterplatten (MTP) mit Flachboden ausgesät. Dabei fand als Kulturmedium MEM Dulbecco (plus 4,5 g/l Glukose plus 3,7g/l NaHCO₃ ohne Glutamin) Verwendung, dem 5 % fötales Kälberserum (FKS) und Penicillin(100 U/ml)/Streptomycin (0,1 mg/ml) zugesetzt waren.

Unmittelbar vor Zugabe der Prüfsubstanzen zur Zellkultur erfolgte ein Austausch des Mediums gegen 100 µl Medium, das anstelle von FKS 1 mg/ml Rinderserumalbumin (BSA) enthielt. Prüfsubstanzen wurden in den gewünschten Konzentrationen dem BSA-haltigen Medium zugesetzt. Als Positivkontrolle wurde TGFβ₁ (Transforming growth factor β₁) in Konzentrationen von 0,1-0,2 ng/ml mitgeführt. Pro (Positiv-) Kontrolle bzw. Substanzkonxentration wurden Dreifachbestimmungen durchgeführt.

Die Inkubation der Zellkulturen mit Prüfsubstanzen erfolgte 24 Stunden lang, in den letzten 3 Stunden zusätzlich unter Anwesenheit der BrdU-Sonde (Zugabe von 10 µl 100 µM 5-bromo-2'-deoxyuridin-Lösung).

Am Ende der Inkubationszeit wurde der Zellrasen mit 200 µl FixDenat™-Lösung bei Raumtemperatur 30 min lang fixiert und gleichzeitig die DNA denaturiert. Anschließend wurde der fixierte Zellrasen mit 100 µl Anti-BrdU-POD-Lösung überschichtet und für 90 min bei Raumtemperatur inkubiert. Nach 3-maligem Waschen mit je 200 µl PBS-Lösung wurden die MTP-Vertiefungen mit 100 µl Substratlösung (TMB=Tetramethylbenzidin) versetzt und 5 min lang bei Raumtemperatur inkubiert.

Die optische Dichte wurde mittels des MTP-Readers ATTC 340 der Firma SLT bestimmt, dabei betrug die Wellenlänge des Meßstrahls 370 nm, die des Referenzstrahls 492 nm. Die Aufnahme und Verrechnung der Rohdaten erfolgte mit der Software 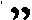 easyWINbasic" der Firma SLT.

Bei der Auswertung dienten Zellkulturen, die ausschließlich BSA-haltiges Medium erhalten hatten, als Kontrollen (100 %).

**Tabelle I**

| Einfluß von L-α-*cis*-9-Octadecensäure-2-hydroxy-3-phosphonooxy-propylester auf die DNA-Synthese-Rate fetaler Rattenosteoblasten | | | | |
|---|---|---|---|---|
| Konzentration (µg/ml) | 0,3 | 1,0 | 3,0 | 10,0 |
| Wirkung in % zur Kontrolle (Kontrolle=100%) | 161 ± 13 | 185 ± 12 | 253 ± 30 | 284 ± 22 |
| Mittelwert ± Standardabweichung, n=8 | | | | |

Verbindungen der Formel (I) wurden ebenfalls in einem in vivo Versuchsmodell in Balb/c Mäusen auf die Stimulation der Knochenbildung untersucht. Die Experimente wurden in Anlehnung an Mackie und Trechsel: Stimulation of bone formation in vivo by transforming growth factor-β: Remodeling of woven bone and lack of inhibition by indomethacin, Bone 11, 295-300, 1990, durchgeführt.

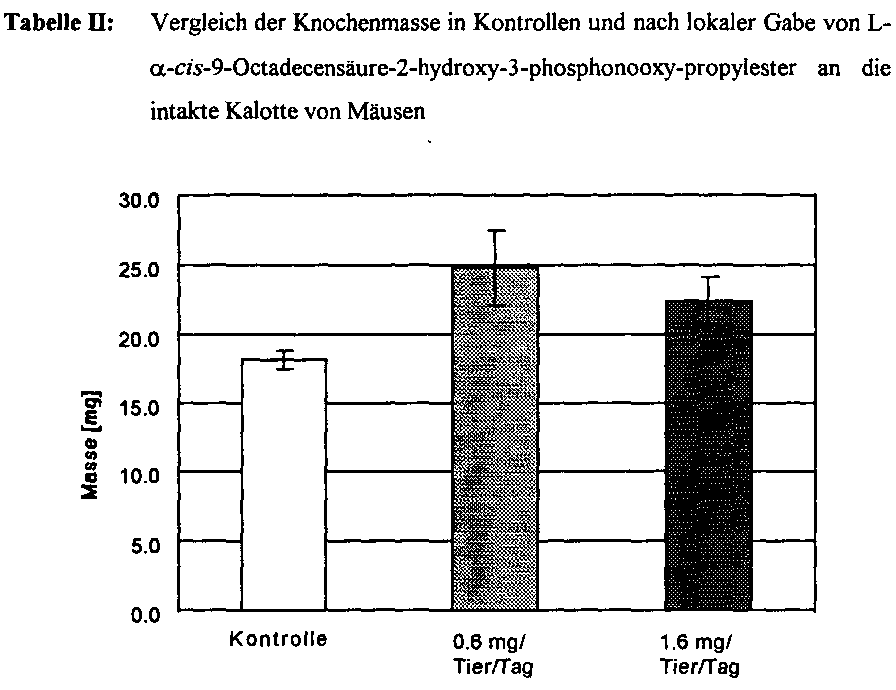

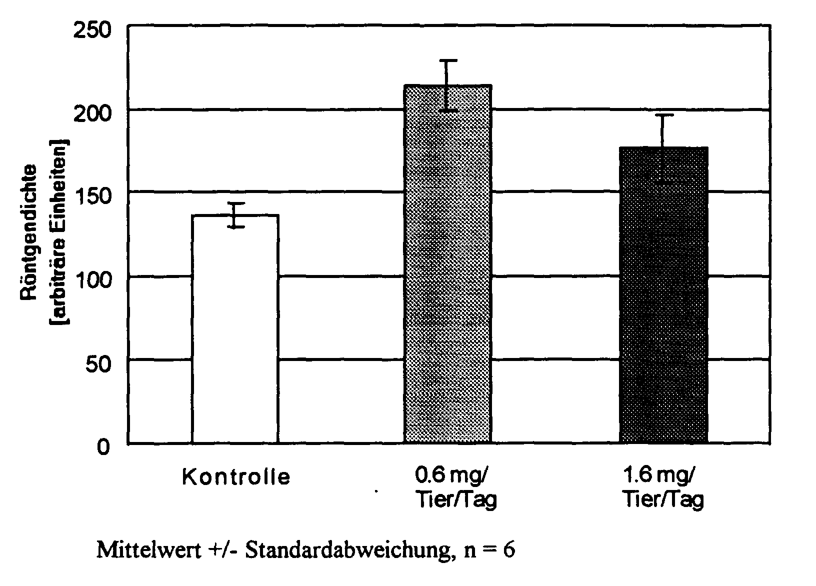

**Tabelle III**

| Zunahme der Knochenmasse in % zur Kontrolle nach lokaler Gabe von L-α-*cis*-9-Octadecensäure-2-hydroxy-3-phosphonooxy-propylester an die intakte Kalotte von Mäusen | | |
|---|---|---|
| | Masse | Röntgendichte |
| 0.6 mg/Tier/Tag | +37 % | +58 % |
| 1.6 mg/Tier/Tag | +23 % | +30 % |
| Mittelwert, n = 6 | | |

## Patentansprüche

1. Verwendung von Lysophosphatidylsäurederivate der allgemeinen Formel (I), 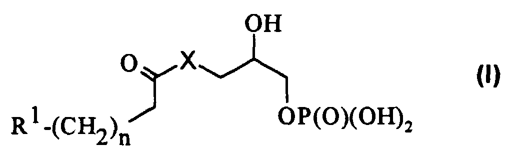 in der
R¹ = Alkyl, Alkenyl oder Alkinyl mit 6 bis 24 Kohlenstoffatomen,
n = 0 - 12
X = Sauerstoff oder NH
bedeuten, sowie deren physiologisch verträgliche Salze, Ester, optisch aktive Formen, Racemate sowie Derivate, die *in vivo* zu Verbindungen der allgemeinen Formel (I) metabolisiert werden können, zur Herstellung von Arzneimitteln zur Behandlung von Knochenstoffwechselstörungen.

2. Verbindungen der Formel (I) 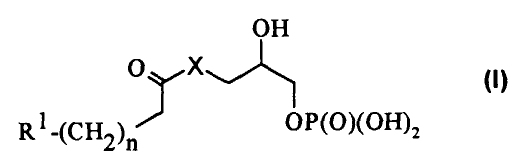 in der
R¹ = Alkyl, Alkenyl oder Alkinyl mit 6 bis 24 Kohlenstoffatomen,
n = 0 - 12
X = Sauerstoff oder NH
bedeuten, mit Ausnahme der Verbindungen (all-*cis*-5,8,11,14)-Eicosatetraensäure-2-hydroxy-3-phosphonooxy-propylester, *cis*-9,*cis*-12-Octadecadien-säure-2-hydroxy-3-phosphonooxy-propylester, (all-*cis*-9,12,15)-Ocatadecatriensäure-2-hydroxy-3-phosphonooxy-propylester oder *cis*-9-Octadecensäure-2-hydroxy-3-phosphonooxy-propylester und mit der Maßgabe, daß wenn X Sauerstoff darstellt, n in der Gruppe - (CH₂)ₙ-CH₃ nicht die Zahl 7,9, 11, 13 oder 15 bedeutet sowie deren physiologisch verträgliche Salze, Ester, optisch aktive Formen, Racemate sowie Derivate, die *in vivo* zu Verbindungen der allgemeinen Formel (I) metabolisiert werden können.

3. Arzneimittel, enthaltend mindestens eine Verbindung der Formel (I), gemäß Anspruch 2, neben üblichen Träger und Hifsstoffen.

4. Verwendung von Verbindungen der Formel (I) gemäß Anspruch 2 zur Herstellung von Arzneimitteln zur Behandlung von Knochenstoffwechselstörungen.

5. Arzneimittel, enthaltend mindestens eine Verbindung der Formel (I), 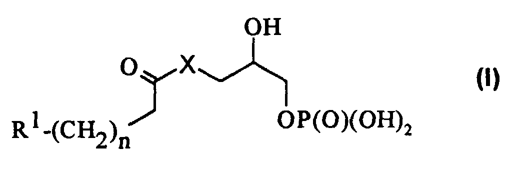 in der
R¹ = Alkyl, Alkenyl oder Alkinyl mit 6 bis 24 Kohlenstoffatomen,
n = 0 - 12
X = Sauerstoff oder NH
bedeuten, mit Ausnahme der Verbindungen (all-*cis*-5,8,11,14)-Eicosatetraensäure-2-hydroxy-3-phosphonooxy-propylester, *cis*-9,*cis*-12-Octadecadien-säure-2-hydroxy-3-phosphonooxy-propylester, (all-*cis*-9,12,15)-Ocatadecatriensäure-2-hydroxy-3-phosphonooxy-propylester oder *cis*-9-Octadecensäure-2-hydroxy-3-phosphonooxypropylester und mit der Maßgabe, daß wenn X Sauerstoff darstellt, n in der Gruppe - (CH₂)ₙ-CH₃ nicht die Zahl 9, 11, 13 oder 15 bedeutet sowie deren physiologisch verträgliche Salze, Ester, optisch aktive Formen, Racemate sowie Derivate, die *in vivo* zu Verbindungen der allgemeinen Formel (I) metabolisiert werden können.

6. Verwendung von Verbindungen der Formel (I) gemäß Anspruch 3 zur Herstellung von Arzneimitteln zur Behandlung von Knochenstoffwechselstörungen.
